# EUROPEAN PATENT APPLICATION

(11) **EP 4 597 427 A1**
(43) Date of publication of application: **06.08.2025**
(21) Application number: 24155193.6
(22) Date of filing: 01.02.2024
(51) Int. Cl.: G06T 7/33, G06T 7/38, G06V 10/771

(54) **METHOD FOR TRACKER-LESS IMAGE REGISTRATION AND SYSTEM FOR CARRYING OUT SAID METHOD**

(71) Applicant: Esaote S.p.A., 16152 Genova (IT)
(72) Inventor: De Beni, Stefano, 16123 Genova (IT); Zini, Luca, 16147 Genova (IT); Guraschi, Nicola, 16142 Genova (IT)
(74) Representative: Bessi, Lorenzo

(57) **Abstract**

A method for tracker-less registering images of the same object generated from two different image datasets, and particularly from two different image datasets acquired by means of two different image acquisition methods and/or techniques comprising:
identifying landmarks in the image or images of the two dataset;
associating to each landmark a univocal semantic description comprising semantic labels describing at least one feature of the landmark and/or geometrical labels describing at least one or some of the geometric relationships between the landmarks identified and/or geometrical labels relating to the shape and/or orientation of the said identified landmarks;
considering two images of the said two data sets as being images along image slicers or image planes registered one with the other when the semantic descriptions related to the landmarks present in the said images are matching one with the other.

## Description

### FIELD OF THE INVENTION

The invention relates to a method for tracker-less registration of images of the same object generated from two different image datasets and particularly from two different image datasets acquired by means of two different image acquisition methods and/or techniques.

The invention also relates to a method for combining images of the same object generated from two different image datasets and particularly from two different image datasets acquired by means of two different image acquisition methods and/or techniques.

In particular the present invention relates to a method for registering and, optionally, combining images generated from an image data set acquired by a real time image data acquisition and image generation process and technique in which images of the same object are generated in a process following the acquisition of the image data, in which images are not generated in real time, this meaning that the said images are not generated essentially at the same time or immediately following the acquisition of the image data set.

In the present disclosure, images which are not generated in real time, in the sense that said images are not generated essentially at the same time or immediately following the acquisition of the image data set, are defined briefly by the term "static images".

On the other hand, images acquired and generated in real time, i.e. images generated during or immediately after the acquisition of the corresponding image data set are briefly defined by the term "dynamic images". In relation to this kind of images, image frames are generated continuously with a certain repetition frequency.

Typical static images are images acquired by imaging techniques like MRI (Magnetic resonance Imaging) or CT (Computer Tomography) or similar techniques for acquiring images and more specifically for acquiring image data of a certain three-dimensional region or three-dimensional Field of View (FOV) of a target body. These examples are not exhaustive and also other volumetric images, i.e. three-dimensional images of a target body which are acquired by ultrasound techniques can be considered "static images" to be combined with two-dimensional images of the same target body acquired by means of ultrasound imaging with high frame rates.

Further imaging techniques providing three-dimensional "static images" are also one of the following non exhaustive examples comprising: Computer Tomography Angiography (CTA), Single-Photon Emission Computed Tomography (SPECT), Positron Emission Tomography (PET)

In a specific application of the present invention, the dynamic images are high frame rate ultrasound images which are two dimensional images acquired along a certain image plane or slice having a certain orientation in a three-dimensional reference system in which also the target object or a region therof corresponding to a selected field of view are placed.

"Static Images" according to the above definition have a high quality in relation to resolution, details, and high Signal to noise Ratio, so that anatomical details are reproduced in a reliable manner and also the artefacts are reduced. Nevertheless, in many applications, high frame rate images are needed, for example in order to follow moving tissues such as, for example, the heart, arteries and/or the lungs or hematic fluxes. Furthermore, when the image acquisition is provided as a monitoring and guiding help for following the positioning of intervention tools, such as for example biopsy needles or other medical tools, a high frame rate of the generated images is required so that it is impossible to follow said tools by using imaging techniques which are configured for generating "static images".

### STATE OF THE ART

The image fusion process between a dynamic modality like US and a static one like MR & CT requires registration of images and tracking of the probe movements to update the image plane of the static modality.

Document IT 102015000024293 discloses a biomedical imaging method for diagnostic evaluation of blood vessels, comprising the following steps:
a) performing a first ultrasound 3D scan, which first scan is synchronized with a detected physiological signal;
b) performing one or more second 3D scans in one or more scan modes, which second scans are synchronized with a detected physiological signal;
c) merging the volumes generated by the first and second scans into a combined volume;
d) performing a third real-time ultrasound 2D scan;
e) registering the third scan with said combined volume.

The real-time image deriving from the third scan, once registered, can be merged with the image data of combined volume along the identical image plane or image slice and the one along which the real-time image has been acquired with the third scan. The merged images can be displayed superimposed one on the other, or no merging is carried out and the real time two-dimensional image and the two-dimensional image, constructed with the image data of the volume along the identical plane or slice identical in position and orientation with the corresponding image plane or slice of the two-dimensional real-time image, are displayed one besides the other. In one embodiment the third scan comprises B-mode imaging allowing to view a real-time image of the region in the FOV. Further imaging techniques can be used as real-time imaging techniques, such as for example Doppler mode imaging or other ultrasound fast or high frame rate imaging modes.

Thus the real time image generated with said third scan further to being used for monitoring dynamic behaviours of structures in the ROI or FOV of the target object under examination, such as particularly in diagnostic images movements and/or changes in time of tissues, organs, hematic fluxes and other time dependent effects, also provides indications of the position and orientation of an image slice or plane along which the said image of the third scan has been acquired in relation to a defined reference system in which the said target object and the said ROI (Region of Interest) and or FOV (Field of View) have a defined position and orientation. These data relating to position and orientation of the said slice or plane are then be used for identifying the position and orientation of the same slice and or plane in relation to the said volumetric image. According to the state of the art this is done by carrying out registration of the image acquired by the said third scan and the said volumetric image. Said registration process defines position and orientation of a slice or plane in the volumetric image corresponding to the position and orientation of the slice or plane of the real time image. Thus, an image can be constructed along the same slice or plane as the real time image by using the image voxels of the volumetric image data falling or contained in the said slice or plane which position and orientation has been determined in relation to the volumetric image.

Many registration techniques are known at the state of the art which are based on more or less complex algorithms which requires a relatively high processing power and time.

Document EP2404551 discloses a method for operating an imaging device for the monitoring of an anatomical region during insertion of metallic objects or objects made at least partly of metal inside the said region. The disclosed device comprises at least a first three-dimensional diagnostic image acquisition means and at least a second two-dimensional image acquisition means. The said first three-dimensional image acquisition means being for example an imaging apparatus such as a TC apparatus, an MRI apparatus, a Fluorangiography apparatus, or similar apparatus which are typically are able to provide "static images" according to the above definition. The second two-dimensional image acquisition means are an ultrasound imaging apparatus. The disclosed device further comprises:
at least a storage unit for storing image data acquired by the first acquisition means and the second acquisition means,
at least a registration and processing unit which acquires two-dimensional images and associates them to three-dimensional images,
at least a means for tracking the displacements of said second two-dimensional diagnostic image acquisition means and at least a display unit. In the said device the second image acquisition means are an ultrasound imaging system comprising a probe and means for driving the probe to emit ultrasound beams and means receiving reflected echoes and for generating images from the said echoes.

Said method for operating the imaging device comprises the following steps:
a) acquiring a three-dimensional image of a specific anatomical district of the body under examination, such as a specific imaging region inside a FOV, by a computed axial tomography device and storing said image data;
b) finding at least a reference point within said anatomical district and
c) acquiring by means of an ultrasound imaging apparatus a two-dimensional image along or at a first section slice or plane of said anatomical district having a position and an orientation so that the said section slice or plane contains the said at least one reference point;
d) identifying said first section plane or slice of the acquired two-dimensional image, within the stored three-dimensional image;
e) constructing a two-dimensional image with data of the three-dimensional image falling along a slice or a plane cutting the said three-dimensional volume and having identical position and orientation as the said first section slice or plane along which the said real-time ultrasound image has been acquired;
f) moving the said slice or section plane along which the two-dimensional real-time ultrasound image has been acquired towards at least a further section slice or plane of said anatomical district having a position and/or orientation different than that of said first section slice or plane of step b) by displacing the ultrasound probe accordingly;
g) tracking the displacement of said ultrasound probe for obtaining the data relatively to position and orientation of the section slice or plane along which said further image has been acquired;
h) identifying within the stored three-dimensional image a further section slice or plane having identical relative position and/or orientation as the section slice or plane at step g) by using the displacement data of the probe determined by the tracking of the displacement of said ultrasound probe;
i) constructing a two-dimensional image with data of the three-dimensional image falling along said further section slice or plane identified at step h);
l) displaying the two-dimensional image obtained at step i) and keeping fixed in place said ultrasound probe for generating images only along the said further section plane or slice;
n) the said section plane or the said slice) along which the real-time two-dimensional ultrasound image is acquired and along which the two-dimensional image is reconstructed from the three-dimensional data being maintained fixed and corresponding to the section plane defined by anatomical markers, while
o) in the displayed combined image deriving from the combination of the two dimensional image which has been reconstructed from the three-dimensional data with the two-dimensional real-time ultrasound image, the contribution of the two-dimensional image reconstructed from the three-dimensional data corresponding to the representation of the anatomical structure along the said slice or section plane and the contribution of the ultrasound image corresponding at least to the representation of the metallic object.

The tracking of ultrasound probes is a well-known technology and many different devices operating according to different physical effects are known in the art. Document EP12183753 discloses a method and an apparatus for ultrasound image acquisition in which image data corresponding to the three-dimensional imaging region or to a three-dimensional field of view of a target body is generated by acquiring a set of 2D images by transmitting an ultrasonic beam into said body and receiving ultrasound echo signals from said body by displacing an ultrasound probe in such a way as to acquire the image data along laterally staggered image slices or image planes, the position of the said image slices or image planes in relation to the target body being determined by means of a probe tracking system. A three-dimensional panoramic image of the said imaging region or of the said FOV of the target body being generated by combining the 2D images of the acquired 3D images based on the information of position and orientation of said probe with respect to said body calculated for each 2D image acquisition. Document EP12183753 further discloses an embodiment of a tracking system of the ultrasound probe capable of determining the position and/or the orientation of the image slice or plane in a reference system.

Known probe tracking systems are usually provided as separate system from the probe which are produced by different producers as the one producing the probes and/or the ultrasound scanner. Furthermore, tracking systems need at least a tracker for receiving or acquiring signals indicating position and/or orientation of the probe in the space and signalling units which needs to be secured to the probe body. Despite possible mistakes in positioning the signalling units on the probe, the tracking system needs additional units in relation to the ones of the ultrasound scanner and this units generates a relatively high increase of costs. The signalling units needs joint elements allowing them to be secured to the probe body in a stable and precise position and these joints need to be configured in a corresponding manner as the probe body, so that differently configured joints may be necessary for securing the signalling units to the different probes. The tracker needs to be positioned at the site at which the target body is placed for carrying out the imaging session and this may disturb the free movements of the staff. Furthermore, the tracking system needs also a processing unit for determining the position and orientation of the image slices or section planes out of the measured data relating to the position and orientation of the probe. As a further drawback, the processing time needed for current tracking systems to determine position and orientation of each section slice or plane along which an image is acquired during normal probe motion while scanning a target body is relatively high. Also relatively high is the time needed for the known systems to identify the section slices or section planes in the three-dimensional volume corresponding to each of the section slices or section planes along which different images are acquired during the swiping motion of the probe along the region of the target body to be examined.

Thus, a need exists to provide methods and systems that allow image fusion in a simpler way.

### SUMMARY OF THE INVENTION

It is an object of embodiments herein to provide improved methods and systems to fuse multimodality images without requiring additional tracking hardware.

A further object of embodiments herein is to speed up the process of determining the image data of a three-dimensional image data set which contributes to generate an image of a target body along a slice or a section plane corresponding to the image slice or section plane along which two-dimensional real time images have been acquired by an ultrasound imaging system, reducing the computational burden of registering each time the image slice or image plane along which the image data has or is being acquired for generating a real-time two-dimensional image with a corresponding slice or plane in the three-dimensional image data set.

In accordance with embodiments herein, devices, computer program products and computer implemented methods are provided for tracker-less registering images of the same object generated from two different image datasets and particularly from two different image datasets acquired by means of two different image acquisition methods and/or techniques, the devices, the program products and methods comprising, under control of one or more computer systems configured with specific executable instructions:
identifying landmarks in the image or images of the two datasets;
associating to each landmark a univocal semantic description comprising semantic labels describing at least one feature of the landmark and/or geometrical labels describing at least one or some of the geometric relationships between the landmarks identified and/or geometrical labels relating to the shape and/or orientation of the said identified landmarks;
considering two images of the said two data sets as being images along image slicers or image planes registered one with the other when the semantic descriptions related to the landmarks present in the said images are matching one with the other.

According to embodiments herein, a method for registering images of the same object generated from two different image datasets and particularly from two different image datasets acquired by means of two different image acquisition methods and/or techniques comprises:
providing an image data set for generating a three-dimensional image of a region of interest in a target body;
processing the image data set with an anatomical landmark detector;
identifying each anatomical landmark and associating to the corresponding image data a semantic description of the anatomical landmark represented by the said image data;
saving the image data corresponding to each anatomical landmark and the associated semantic description of the corresponding anatomical landmark;
acquiring a first two-dimensional, real time ultrasound image or a first sequence of two-dimensional, real-time images along at least one section slice or plane of the same target body, the said image slice or image plane intersecting the same region of interest of the said target body as at step a);
processing the said first two-dimensional, real-time image data or the said first sequence of real-time two-dimensional image data with the said anatomical tracker for identifying anatomical landmarks within the said two-dimensional image data or the said sequence of two-dimensional image data acquired at steps e);
identifying each anatomical landmark within the said two-dimensional image data or within the said sequence of image data and associating to the corresponding image data a semantic description of the anatomical landmark represented by the said image data;
selecting at least some of the identified anatomical landmarks and the corresponding image data representing the said selected anatomical landmarks and determining a geometrical relationship between the said selected anatomical landmarks and the image slice or image plane along which the two-dimensional real-time image or the said sequence of two-dimensional real-time image has been acquired and in which the said selected anatomical landmarks has been identified;
   i) determining the image slice or the image plane intersecting the said three dimensional image which corresponds to the said image slice or the said image plane along which the said two-dimensional real-time image has been acquired at step e) by choosing the image slice or the image plane intersecting the said three-dimensional image data having the closest semantic description to the one associated at step g) to the image slice or the image plane along which the said first two-dimensional, real-time image or the said first sequence two-dimensional real-time images has been acquired at step e);
   j) selecting the image data of the three-dimensional image data set falling on the image slice or image plane intersecting the said three-dimensional image data set determined at step i) and generating a two-dimensional image of the target body using the said selected image data.

According to a further feature, the position and orientation of a two-dimensional image slice or plane is defined by said selected one or more predefined landmarks which are present in the image along the said image slice or image plane and by the geometric relationship between the said selected landmarks.

According to a further feature, the orientation of the said two-dimensional image slice or image plane may also be improved or refined by additionally determining the geometrical shape of the said selected landmarks.

Once the image data has been determined which are on the image slice or the image plane intersecting the three-dimensional image data set and corresponding to the image slice or the image plane along which the said two-dimensional real-time image has been acquired according to the previous step j) and the corresponding image is generated out of the said selected image data of the three-dimensional image data set, further steps may be carried out consisting in combining the two dimensional image data selected at step j) and the image data of the two-dimensional real-time ultrasound image for generating a combined image, which is then displayed on a monitor and/or printed and/or saved.

Combination can be carried out by many different image combination or fusion algorithms which are currently available at the state of the art.

Furthermore, combining the images can be carried out by combining the two images generated respectively at step j) and at step e) or the corresponding image data from which the said images are generated.

A further alternative for combining the image data can be to combine the raw data acquired by the ultrasound system and the imaging system by which the three-dimensional image data has been acquired.

The above alternatives may be provided together so that the possibility is given to choose among them.

According to a further embodiment, for each two-dimensional image slice or plane, a descriptor of the position and orientation of the said image slice or plane relatively to the target body or to a ROI or FOV of the said target body is generated and univocally associated to said two-dimensional image slice or plane. Said descriptor comprises the list of the denominations of the selected landmarks and/or the list of their geometrical relationship and/or their geometrical shapes in the image slice or plane. The descriptors of the image slice or image plane along which each of the real time "dynamic" images are acquired are compared with the descriptors of two-dimensional image slices or image planes intersecting said three-dimensional "static" image or a ROI or a FOV of the said three-dimensional "static" image.

Said descriptors comprise the list of the denominations of the selected landmarks present on each one of the said image slices or planes intersecting the three-dimensional "static" image and the list of their geometrical relationship and/or their geometrical shapes in the corresponding image slice or plane;

The image slice or image plane intersecting the three-dimensional image or a ROI or a FOV thereof corresponds to the position and orientation of the image slice or the image plane along which said two-dimensional real-time "dynamic" image is acquired when the descriptor univocally related to the image slice or image plane intersecting the said three-dimensional "static" image or a ROI or a FOV of it corresponds to the descriptor of the image slice or the image plane of the said two-dimensional real-time "dynamic" image.

The step of comparing the descriptors of an image slice or an image plane of a two-dimensional, real-time "dynamic" image with the descriptors of image slices and image planes intersecting the said three-dimensional, "static" image may be carried out by means of different kind of algorithms.

According to a preferred embodiment such a step of comparing is carried out by applying a matching algorithm such as a correlation algorithm or a machine learning algorithm such as a classification algorithm.

As it will appear more clearly from the following description of a detailed embodiment, the use of semantic descriptors, alone or together with geometric relationships between the selected landmarks and/or the geometric shapes of the said selected landmarks, reduces the number of data to be processed for carrying out the matching between the image slice or image plane along which a two-dimensional real-time "dynamic " image has been acquired and the corresponding image slice or image plane intersecting a three-dimensional "static" image. This allows to spare processing power and processing time so that image registration can be carried out rapidly allowing to have high frame rates avoiding high performance hardware for supporting the image slice or plane matching.

Furthermore, the process is not limited to the use of machine learning algorithms but can also be carried out using different analytical or statistical algorithms which do not require training steps. These steps often need a high number of training data and the algorithm fitness in relation to the quality of the output is strongly related to the quality of the training data sets and to the specific training process chosen for carrying out the training step. Nevertheless, in carrying out the image slice or image plane matching by means of the above-described semantic descriptors also machine learning algorithms can be applied and are within the scope of protection of the claimed combinations.

Embodiments also relate to a system for registering images of the same object generated from two different image datasets and particularly from two different image datasets acquired by means of two different image acquisition methods and/or techniques which system is configured for carrying out the method of registering images according to one or more of the embodiments and variants disclosed above and optionally also in the following detailed description.

According to an embodiment, said system comprises:
at least a processing comprising at least a processor, at least one memory, a data input interface and a data output interface, a scanner for acquiring real-time ("dynamic") two-dimensional images of a target object or of a ROI or a FOV thereof. The three-dimensional image data of said target object can be provided through the input interface while two-dimensional, real-time image data are acquired by the said scanner which is in communication with the processing unit;
a processing algorithm providing the instructions for making said at least one processing unit able to carry out the processing steps of the method according to embodiments herein for registering at least one or some or each of the said real-time, two-dimensional images with an image generated along an image slice or plane intersecting the said three-dimensional image and which position and orientation relatively to the target object or a ROI or a FOV thereof corresponds to the position and orientation of the image slice or image plane along which a corresponding one of the said two-dimensional real-time images has been acquired.

According to a further feature, said system further comprises an image combination or fusion processor comprising a processing unit and a memory in which an image combination and/or fusion program is stored or may be stored, said combination and/or fusion program comprising the instructions for the processing unit which, when executed, render said processing unit able to carry out the steps of combining and or fusing a real-time two-dimensional image acquired by the said scanner with a two-dimensional image generated with the contribution of the image data of the said three-dimensional image falling or contained in an image slice or on an image plane which position and orientation in relation to the target object or a ROI or a FOV thereof corresponds to the position and orientation of the image slice or the image plane along which the said real-time two-dimensional image has been acquired by the said scanner.

Said processing unit of the image combination or fusion processor can be an additional processing unit specifically dedicated and configured for carrying out the image combination or fusions steps or the same processing unit for carrying out the registering and matching steps.

According to a further feature, the system comprises at least one display and a display processor which comprises the instructions for displaying on the display the images acquired by the said scanner and the images generated from the said three-dimensional image along an image slice or image plane which position and orientation in relation to the target object or a ROI or a FOV thereof corresponds to the position and orientation of the image slice or the image plane along which the said real-time two-dimensional image has been acquired by the said scanner;
said display instructions being configured for alternatively displaying the said two images separated one form the other or one beside the other or the said two images overlaid and/or combined and/or fused in an image comprising the image information of both the said images.

According to a further feature, the scanner for acquiring real-time, two-dimensional images is an ultrasound imaging system.

Still according to a further embodiment, the system is an imaging system for acquiring images of a target object and in which there is integrated a system for registering images of the same object generated from two different image datasets one of which datasets being the image data acquired by the said imaging system and/or also a system for combining or fusing the registered or matched two-dimensional images acquired and generated in real time (so called dynamic images) and the images generated with the contributions of the three-dimensional image (so called static image) falling or contained in the image slice or the image plane having matching orientations and positions relatively to the target object or a ROI of it or a FOV as the image slice or image plane along which the said two-dimensional real-rime images has been acquired.

According to an embodiment, said imaging system is an ultrasound imaging system for acquiring real-time two-dimensional images, particularly of the digital kind.

Further improvements of the invention will form the subject of the dependent claims.

### BRIEF DESCRIPTION OF THE DRAWINGS

The characteristics of the invention and the advantages derived therefrom will be more apparent from the following description of non-limiting embodiments, illustrated in the annexed drawings, in which:
Figure 1 is a high-level diagram of a system according to an embodiment herein.
Figure 2 is a block diagram of a generic processing hardware which is provided in combination with a software comprising the instructions for the processing hardware which, when executed, renders said processing hardware capable of carrying out the steps of an embodiment of the method for combining images of the same object generated from two different image datasets and particularly from two different image datasets acquired by means of two different image acquisition methods and/or techniques.
Figure 3 diagrammatically shows process steps of an embodiment of the method for combining images of the same object generated from two different image datasets and particularly from two different image datasets acquired by means of two different image acquisition methods and/or techniques.
Figure 4 is a block diagram showing matching the image slices or image planes by semantic registration.
Figure 5 is a flowchart showing the workflow for acquiring three-dimensional "static" three-dimensional images of a target body or of a ROI or a FOV thereof and for classifying the landmarks contained in said three-dimensional images.
Figure 6 is a flowchart showing the workflow of the method steps of an embodiment of the method and relating to steps of acquiring the two-dimensional "dynamic" image of a target body or of a ROI or a FOV thereof and of classifying the landmarks contained in the said two-dimensional image and carrying out the image slice or image plane registration and/or additional image combination or fusion.

### DETAILED DESCRIPTION OF A PREFERRED EMBODIMENT

In the present specification and in the claims, the generic term "image" in relation to two-dimensional or three-dimensional images of a target body has to intended as comprising, if not specifically otherwise indicated, the physical raw data acquired by a scanner or a probe in applying an imaging technique on a target body, and or the image data comprising the contribution of the raw data to the generation of an image and extracted from the said raw data by processing it and the image intended as the pixels or voxels forming a two dimensional or a three-dimensional array which pixels or voxels are identified by their relative position and by values of parameters defining their appearance in an image printed on a display or on a substrate such as paper or a film.

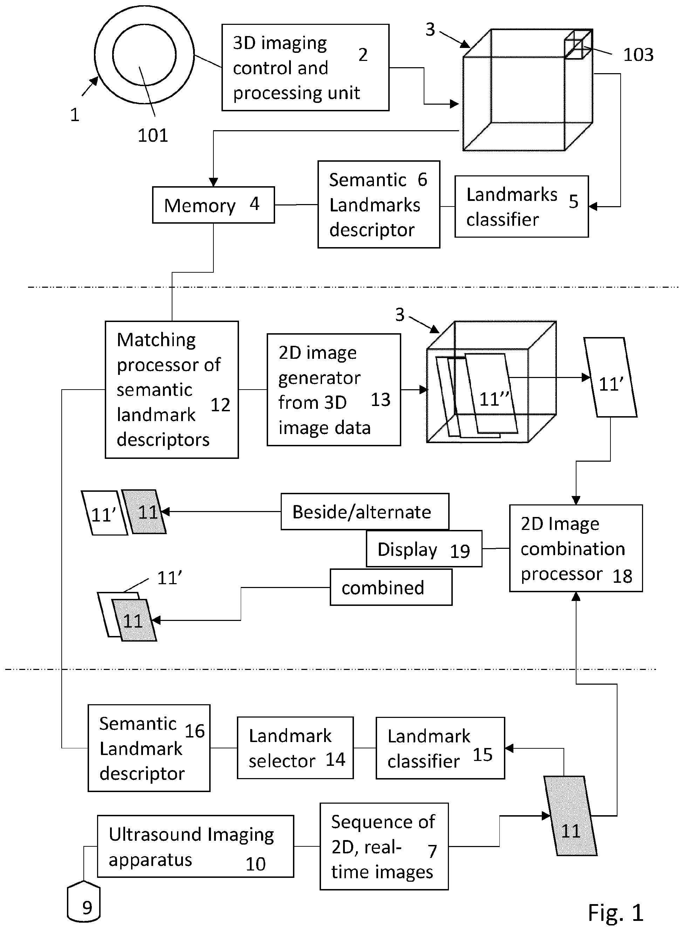

Figure 1 shows a high-level block diagram of an embodiment of a system according to the present invention which is configured for carrying out embodiments of the method according to the present invention.

In figure 1, a part of the system is configured to carry out a first imaging session of a target body or of a ROI of it or in a FOV within the said target body or ROI. This first imaging session is carried out in a different time, preferably at an earlier time than a following second imaging session of the same target body and or of the same ROI or FOV of it. The imaging is carried out by using an image acquisition system operating according to an imaging technique which allows to acquire three dimensional images having a high quality, namely high resolution, low signal to noise ratio, reduced presence of artifacts, etc., etc.. Normally these imaging systems and techniques require long time for scanning a three-dimensional ROI or FOV of a target object and for reconstruction image data and an image from the acquired raw signals. As already disclosed above non exhaustive examples of such kind of imaging techniques and the corresponding imaging systema are Magnetic Resonance Imaging (MRI), Computer Tomography (CT), Computer Tomography Angiography (CTA), Single-photon emission computed tomography (SPECT), Positron emission tomography (PET) and other imaging systems and techniques for acquiring high-quality three-dimensional images.

In fig. 1 this kind of imaging system is represented by a scanner 1 having a gantry 101 and collecting signals from the target object in the gantry of the scanner 1 which signals contains information from which image data can be extracted allowing to generate an image corresponding to the target object, a ROI of it or a FOV of it. The three-dimensional imaging apparatus is further provided with a control and image processing unit globally represented by the box 2 in figure 1. The configuration of this control and image processing unit depends on the kind of imaging technique which the imaging apparatus is configured to carry out and also to the specific configuration of the apparatus itself. As a result of an imaging session, the three-dimensional imaging apparatus generates a three-dimensional array of image data from which a three-dimensional image 3 can be reconstructed, typically in the form of an array of voxels 103. The above acquired physical signals which in the present specification are defined as raw data and/or the image data extracted from said raw data and/or the reconstructed image may be stored in a memory 4. This memory 4 can be a memory internal to the imaging apparatus and/or a memory of a further processing unit, such as a processing unit for detecting, identifying and labelling characteristic landmarks which are present in the acquired image that will be disclosed in the following, or a portable memory in combination with a write/read interface of the said memory connected to the imaging apparatus, or a combination of the above listed alternatives.

In the present embodiment and without being a limitation, the three-dimensional image in the form of the corresponding raw data and/or of the corresponding image data or of the corresponding array of voxels is subjected to a landmark classifier 5. This landmark classifier 5 may be configured in many different forms and in one embodiment the said classifier comprises a processing unit having a memory in which a landmark classification algorithm is stored and which classification algorithm comprises instructions which when executed by the processing unit of the classifier 5 renders the said processing unit able to identify raw data and/or image data and/or image voxels as relating to a specific landmark. All the raw data, image data or image voxels relating to the three-dimensional image is subjected to processing by the classifier and each landmark present in said three-dimensional image is identified and said data is univocally associated to each landmark. A semantic landmark descriptor 6 is provided allowing to evaluate each landmark and associate to it, i.e. to the corresponding raw data and/o image data and/or image pixels or voxels classified as representing a specific landmark, a semantic description of the landmark.

The Semantic landmark descriptor can be configured in many different ways.

In an embodiment, the analysis of the identified landmarks in the three-dimensional image is caried out manually by a user and said descriptor 6 may be in the form of a processing unit, having a processor, at least one memory, a display, a user interface comprising one or more input means for data, and/or commands by a human user. The user-interface is configured for allowing the user to display the part of the three-dimensional image relating to a landmark and for entering semantic descriptive labels and univocally correlating the said labels to the image part representing a landmark. Once each landmark is univocally associated with a corresponding semantic description a database may be generated in which the raw data and/or the image data and/or the pixels or voxels corresponding to each one of the landmarks identified in the three-dimensional image is stored in combination to the semantic labels.

As a further improvement, further data can be added to the semantic labels associated to each landmark, the said data may be the geometric relation to other landmarks identified in the three-dimensional image and/or also data about the geometric shape of the corresponding landmark. Also these data may be univocally associated to each landmark together with the semantic labels.

According to a variant embodiment, the process of determining the semantic labels describing the landmarks and/or the geometric relationships between the landmarks and/or the geometric shapes of the landmarks may be carried out automatically, such as by means of the same classification algorithm and/or by other image processing algorithms.

Both variant embodiments may be carried out by a processing unit, which may be a separated one form the processing unit forming the landmark classifier or which processing unit may be the same one of the landmark classifiers. In both cases a semantic landmark descriptor software is loaded in a memory of the processing unit and is executed by said processing unit, which software comprises the instructions which renders the processing unit able to carry out the tasks for the manual and/or automatic semantic label generator and/or the tasks for determining in a manual and/or automatic way the geometric relationships between landmarks and/or geometric shape of the landmarks and also the steps of univocally associating the landmarks (raw data and/or image data and/or voxels related to each landmark) and the semantic labels and/or the geometric relationships to the other landmarks and/or the geometric shapes of the landmarks.

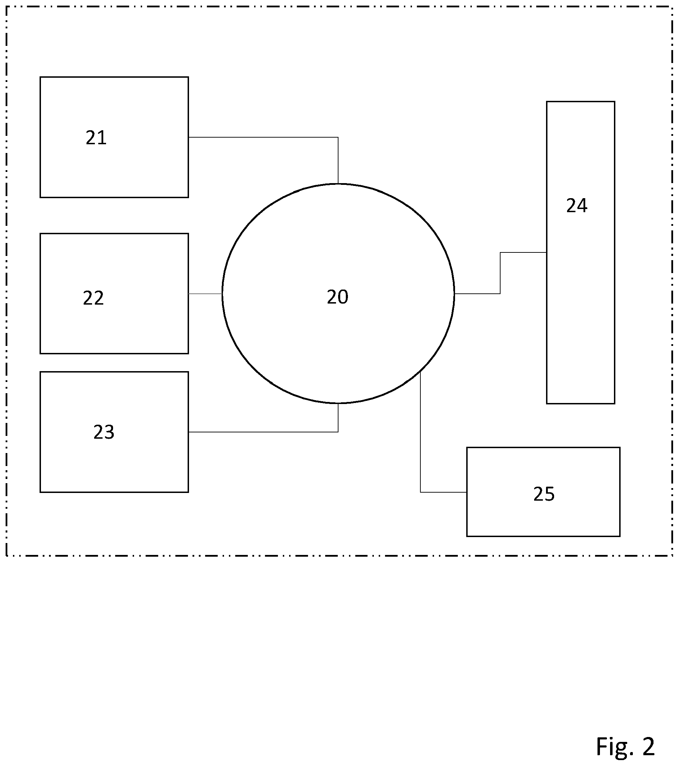

In figure 2 a high-level block diagram of a generic processing unit is shown which can be used as a control and processing unit 2 of the imaging apparatus and/or as a landmark classifier 5 and/or as a semantic landmark descriptor 6. The processing unit comprises a processor or a CPU 20 to which at least one memory 21 is connected. The processing unit may further comprise at least one display 22 and at least one user interface 23. The said one or more user interfaces summarized by the box 23 are intended to represent any kind of human interface with the processing unit 20 either for inputting data or commands or for data output additionally to the display 22.

With the block identified by 24, further interfaces are described such as, for example, interfaces with other devices or processing units. These interfaces may be of the kind using wired or non-wired, e.g. wi-fi, connections. Furthermore with 25 an optional read/write device for a portable memory device is indicated, such as an USB interface for connecting an external memory and/or a memory support read/write unit operating in combination with memory disk such as CD-Rom and/or DVD-Rom or CD-Ram or DVD-Ram or similar memory supports. A software containing the instructions for the processor 20 which render the processor able to carry out the steps of controlling the imaging apparatus and/or of processing the acquired signals in order to generate a three-dimensional image and/or the analysis of the image for detecting the landmarks and the definition of the semantic labels and of the geometric features may be saved in the memory 21 and executed when the corresponding tasks has to be carried out by the processing unit.

As already described, only one processing unit may be provided which carry out each of the above disclosed tasks, or at least some of the tasks such as controlling the imaging apparatus and/or processing the acquired signals for generating an image and/or detecting and classifying the landmarks and/or determining the semantic labels and the geometric labels and univocally associating to a landmark a semantic descriptor comprising the said semantic labels and/or the said geometric labels is carried out by two or more processing units each one configured for carrying out one or more of the above tasks.

In relation to the above described process, it has to be noticed that this process, providing at least one three-dimensional image, detecting and classifying every landmark or a group of landmarks selected according to predefined criteria, analysing each of the landmarks and determining semantic labels describing the said landmarks and geometric labels describing their geometric relationship to other landmarks or the shape of a corresponding landmark and finally generating a database in which the groups of raw data and/or of image data and/or of image voxels representing in the three-dimensional image respectively each of the identified landmark are univocally associated to a semantic descriptor comprising semantic labels and/or geometric labels, is carried out "off-line" i.e. non in real time with the further steps of the method according to the present invention. Normally the above steps are carried out at an earlier time and the above indicated database is used for carrying out the further steps of the method by the disclosed system dedicated to carry out the said method.

The term "off-line" is used in the present description for summarizing the fact that the process steps are not carried out at the same time, i.e. in real-time, with the further steps disclosed in the following, but said process steps relating to the acquisition and processing of the three-dimensional image are carried out and terminated with the generation of the database comprising the groups of raw data and/or of image data and/or of image voxels representing in the three-dimensional image respectively each of the identified landmarks univocally associated to a landmark specific semantic descriptor comprising optionally also semantic labels and/or geometric labels.

A dotted line separates graphically said "off-line" process steps from the so called "on-line" steps.

At least one two-dimensional, real-time image, or a sequence of said two-dimensional real-time images 7, here defined as "dynamic images" is acquired by means of an ultrasound probe 8 of an ultrasound imaging apparatus 10. Each two-dimensional, real-time image is acquired along an image slice or an image plane having a certain position and a certain orientation relatively to the target body and/or a ROI of it and or a FOV as graphically represented by the slice or plane 11.

During the imaging session the raw data is acquired along different image slices or image planes having different positions and orientations relatively to the said target object and/or a ROI of it and the corresponding image along each one of the said slices and/or planes are immediately generated a sequence of images generated and subjected to processing by a landmark classifier 15. The identification of the landmarks present in each two-dimensional real-time image is carried out immediately after the image data has been made available and/o the image has been generated and for each landmark identified in each image a semantic landmark descriptor 16 is carried out analysing the landmarks, providing for each of the landmarks identified specific semantic labels and providing optionally also specific geometric labels. With semantic labels identical semantic labels are meant as described above. This is also true for the geometric labels which can be geometric relationships between landmarks identified in a two-dimensional image and/or geometric shape descriptions of the said landmarks the said geometric labels being of the same kind as the ones described above in relation to the landmarks identified in the three-dimensional image.

For each two-dimensional image the semantic descriptors comprising the semantic labels and/or the geometric labels specific to each or at least some of the landmarks identified in the two-dimensional image are univocally associated with the position and/or orientation of the image slice or the image plane along which the two-dimensional image has been acquired.

The data relating to the semantic descriptor of each two-dimensional image of a sequence of two-dimensional images is fed to a matching processor 12, in which a matching is carried out between the semantic descriptions associated to the landmarks identified in a two-dimensional real-time image 7 and the semantic descriptions of the landmarks identified in the three-dimensional image 3 along each slices or planes 11" intersecting the said three-dimensional image 3. When a matching is found among the said semantic descriptions, the system sets the image slice 11' or the image plane intersecting the three-dimensional volume 3 for which a matching has been determined with the semantic descriptions of the landmarks in the two-dimensional real-time image 7 as the image slice or the image plane 11' corresponding to the one 11 of the two-dimensional, real-time image/s 7. A two-dimensional image is generated by a two-dimensional image generator 13 using the raw data, and/or the image data and/or the voxels falling on the said image slice or image plane 11' and the said image is considered being registered with the two-dimensional real-time image/s 7.

According to an embodiment, a landmark selector 14 can be provided which is configured to allow the user to select only some of the landmarks identified and to limit the definition of the semantic descriptions only relatively to the said selected landmarks and comprising the said semantic labels and/or the said geometric labels relative to the said selected landmarks.

The landmark selector can be provided as shown in figure 1 in the landmark identification proc ess related to the two-dimensional real-time images 7, preferably before the step of generating the semantic descriptors of the identified landmarks by the descriptor 16 and/or also in the off-line or previous process of identifying the landmarks in the three-dimensional image 3 by the classifier 5 and preferably before the step of generating the landmark descriptions by the landmark descriptor unit 6.

The selector can be configured as a manual unit. This unit can be provided as a selector program which can be executed by a dedicated processing unit or by the control and processing unit 2 of the imaging apparatus and/or by a processing unit specifically dedicated to work as a landmark classifier 5 and/or 15 and/or as a semantic landmark descriptor 6 and/or 16. The said selector program comprises the instructions which when executed by the processor 20 renders the said processing unit able to receive a command for launching the execution of the selector program, displays the images highlighting in it the identified landmarks and allow the user to navigate each of the said identified landmarks in order to select one or more of all of the said landmarks. The selected landmarks, i.e. the raw data, and/or the image data and/or the pixels or voxels of the image representing the said landmarks in the image are then labeled as landmarks to be subjected to the step of generating a univocally related semantic description by the semantic landmark descriptor 6/16.

In a variant embodiment, the selection can be carried out automatically. In an embodiment, the automatic selection can be carried out by defining a list of landmark classes potentially present in the target object and/or in a ROI of it and/or in the FOV of the imaging apparatus and which list is compared by a matching algorithm with the landmarks identified in the acquired image. Only the landmarks identified in the image and matching with one of the landmarks in the list are then further processed by the landmark semantic descriptor 6 or 16 (depending on the configuration of the system according to the above disclosed alternative embodiments).

The two-dimensional image generated along the image slice or plane 11' whose semantic description is matching with the semantic description of the two-dimensional, real-time image/s 7 acquired along the image slice or image plane 11 can be fed to a 2D image combination processor 18 together with the two-dimensional real-time image 7 and the said two-dimensional images are combined together.

The two images can be combined according to many alternative combination functions or processes such as, for example and without limitation, addition of the image contributions of each image and/or image fusion and other combination algorithms. The combination can be carried out by operating with the raw data acquired by the imaging systems and/or the image data and/or the data relating to the definition of the appearance and position of pixels or voxels of the image reconstructed from the said raw data or from the said image data.

The two-dimensional images along each slice or plane 11 and 11' having matching semantic descriptions and/or the combined image generated from the said images can be stored in a memory. Furthermore, the said two-dimensional images along each slice or plane 11 and 11' having matching semantic descriptions can be displayed on a display 19 separated and one beside the other and/or the combined image generated from the said images can be displayed on the display 19. A user interface may have means for inputting a command for changing the display of the images form the separate display of the images one beside the other to the display of the combined image or to a display of the combined image and beside it one of the said two-dimensional images along the slice or plane 11 and/or 11'.

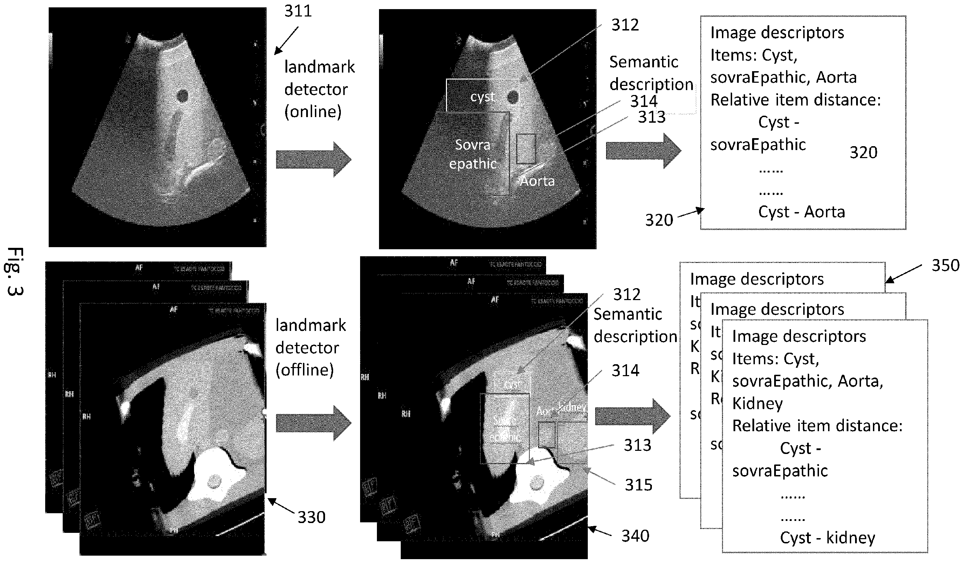

Figure 3 shows with some practical examples the steps of the method according to the above description and executed by the embodiment of the system for carrying out the said method disclosed above.

The upper part of the figure 3 shows the results of the steps discloses above and carried out in the so called on-line or real-time part of the process. 311 shows an example of a two-dimensional, real-time image acquired by an ultrasound system such as the one indicated with 10 in figure 1. The said two-dimensional, real-time image is acquired along an image slice or an image plane as the one defined by 11 in relation to figure 1.

The said image or the related image data or the related raw data are processed by a landmark detector, such as a classifier 15 of figure 1. The boxes 312, 313 and 314 show three landmarks which has been detected in the said image 311.

Each of the said landmarks 312, 313, 314 is subjected to processing by a landmark descriptor which can be configured according to one of the above disclosed alternatives for either generating manually by a user a semantic label of the object represented as a landmark or for generating these semantic labels in an automatic way.

According to a further feature, geometric labels can be added to each semantic label. The geometric label may comprise geometric relationships between the landmarks identified in the image and/or also geometric features of the shapes and orientation of the said shapes of the objects being identified as landmarks.

Number 320 identifies an example of a semantic description of the landmarks detected in the image 311. As semantic labels the term "Cyst" and "sovra-epathic and Aorta" are recorded and as a geometric label the distance between the Cyst and the sovra-epatic and the distance between the cyst and the aorta 314 are measured in the image and recorded in the semantic image description 320.

Number 330 indicates a series of two-dimensional images taken along several different image slices or image planes and forming a three-dimensional image. The three-dimensional image is subjected to processing by a landmark detector 5. As disclosed above in relation to figure 1, the process is generally carried out "off-line", this means at an earlier time that the process of the acquisition of the two-dimensional, real-time images and of the landmark detection and landmark description as disclosed.

As a result of the processing of the image 330 by the landmark detector, such as the landmark detector 5 of figure 1, each detected landmark in the three-dimensional image is identified as graphically represented by the images indicated with 340 in which the landmarks related to a Cyst 312, to the sovra-epathic 312, to the Aorta 314 and to the kidney 315 are shown. The identified landmarks are further subjected to the step of generating semantic descriptions by a semantic descriptor generator as the one indicated by number 6 in figure 1. To each landmark in a semantic label is associated and also a geometric label is associated. This is shown in the semantic descriptions indicated by 350.

It has to be noticed, that without any limitation, the three-dimensional image may be formed by combining a plurality of two-dimensional images taken along a series of adjacent image slices or image planes. This is represented by the images indicated by numerals 330, 340 in figure 3. In this case the process of identifying the landmarks and of determining and associating the semantic labels and/or the geometric labels to the said landmarks may be carried out for the image related to every slice or plane of the said series of adjacent image slices or planes by which the three-dimensional image has been generated.

The above embodiment is not to be considered limitative, but only a possible example on how a three-dimensional image can be acquired and constructed and further on how the said image can be subjected to landmark identification and to the association of semantic descriptions to each landmark.

Differently as the shown example, the three-dimensional image can be considered as a unitary three-dimensional array of image voxels and the steps of identifying the landmarks, generating the semantic descriptions and the geometric description of the landmarks may be carried out in relation to the entire three-dimensional image.

In this case the process of detecting the landmarks and of generating the semantic and geometric descriptions may be carried out by suing machine learning algorithms which may simplify the processing of three-dimensional arrays of data.

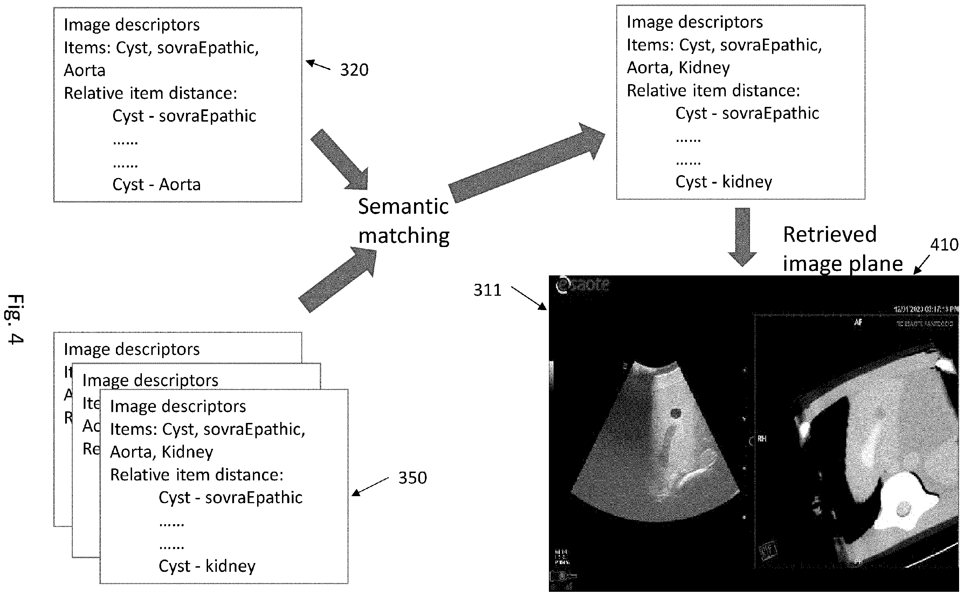

Figure 4 shows a simplified embodiment of the step of registering image slices or image planes of the two-dimensional real-time images with an image slice or an image plane of an image reconstructed from the three-dimensional image. The registration step is carried out by subjecting to a matching process of the semantic description of the landmarks identified in the two-dimensional, real-time image with the semantic description of a slice or a plane intersecting the target object, i.e. the three-dimensional image reproducing at least a ROI or an image in a FOV of the said target object. The semantic description comprising both semantic labels and geometric labels as define above according to the different alternatives disclosed.

The example represented in figure 4 is applied to the two-dimensional, real-time ultrasound image acquired along a certain slice or section plane as shown by numerals 311 and to the three-dimensional image 330 formed by a combination of two-dimensional images along adjacent slices or planes. The semantic description 320 univocally associated to the image slice or image plane along which the two-dimensional, real-time image has been acquired is matched with the semantic description of a slice or a plane intersecting the three-dimensional image of the target body and the image slice or image plane corresponding to the semantic description matching with the semantic description of the two-dimensional real-time image is defined as the image slice or image plane being in register with the image slice or the image plane along which the said two-dimensional, real-time image has been acquired. As indicated by 410, the image along the said image slice or image plane intersecting the three-dimensional image and having a semantic description matching with the one 320 of the two-dimensional real-time image 311 is then retrieved by reconstruction said image using the raw data contributions and/or the image data and/or the voxels falling in or on the said image slice or the said image plane.

The said image indicated by 410 may be combined with the two-dimensional, real time image and displayed as already disclosed above or the said two-dimensional image 410 can be displayed beside the two dimensional-real time image 311 as indicated in figure 4.

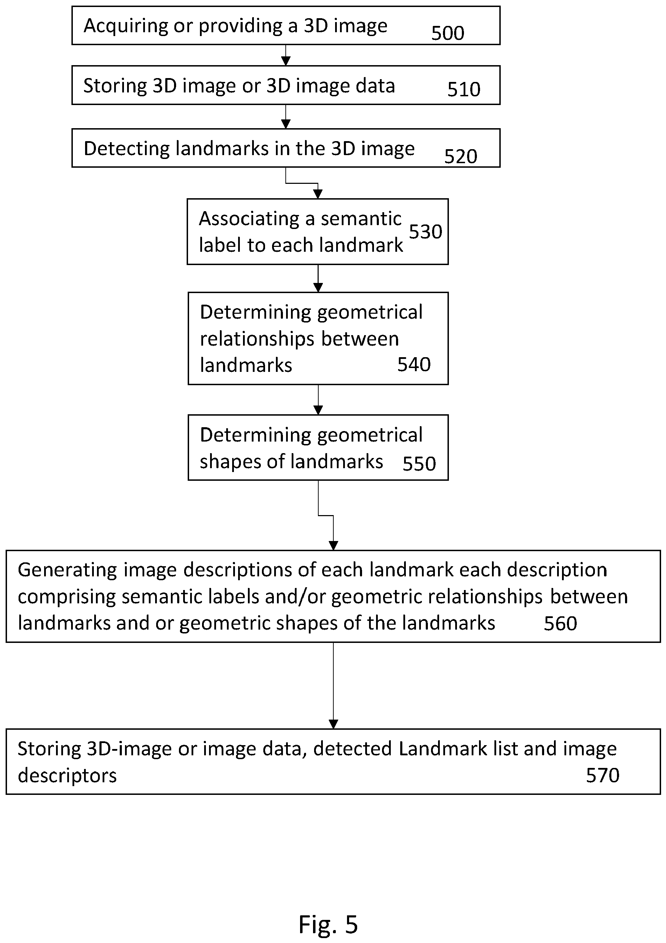

Figure 5 is a representation of an embodiment of a workflow of the process for carrying out the off-line steps of the method according to the present invention according to one or more of the embodiments disclosed above and according to the meaning defined above for the term "off-line" steps.

At first time a step 500 of acquiring a three-dimensional image is carried out, for example, by an imaging method which is configured for high quality, for example high resolution and/or high signal to noise ratio, etc. images, like one or more of the imaging methods and techniques disclosed above and used for acquiring the said "static images".

The three-dimensional image, in the form of the corresponding raw data and/or the corresponding image data and/or the corresponding reconstructed image, i.e. the parameters describing position and aspect of the pixels or voxels of the said image may be saved as indicated by 520. At step 530, the said three-dimensional image, in one or more of the data representing the said three-dimensional image is subjected to a process for detecting in the said image one or more landmarks. Each one or a selected ones of the said landmarks are then subjected to determining semantic labels univocally defining the said landmarks and to associating the said semantic labels to the corresponding landmark as indicated at step 540. An optional further step 550 of determining geometric labels of the said landmarks is carried out. One first kind of geometric labels can be the values of the distance of a certain landmark from the other landmarks identified in the image or from part of them. Optionally additional geometric labels can be generated and univocally associated to a landmark, which labels consist in geometric descriptions of the shape and/or of the orientation in space of the said shape of the said landmarks as indicated by step 560.

The following step 570 provides for generating image descriptions of the said landmarks said descriptions comprising semantic labels and/or geometric relationships between landmarks and or geometric shapes of the landmarks and associating to each landmark a corresponding image description.

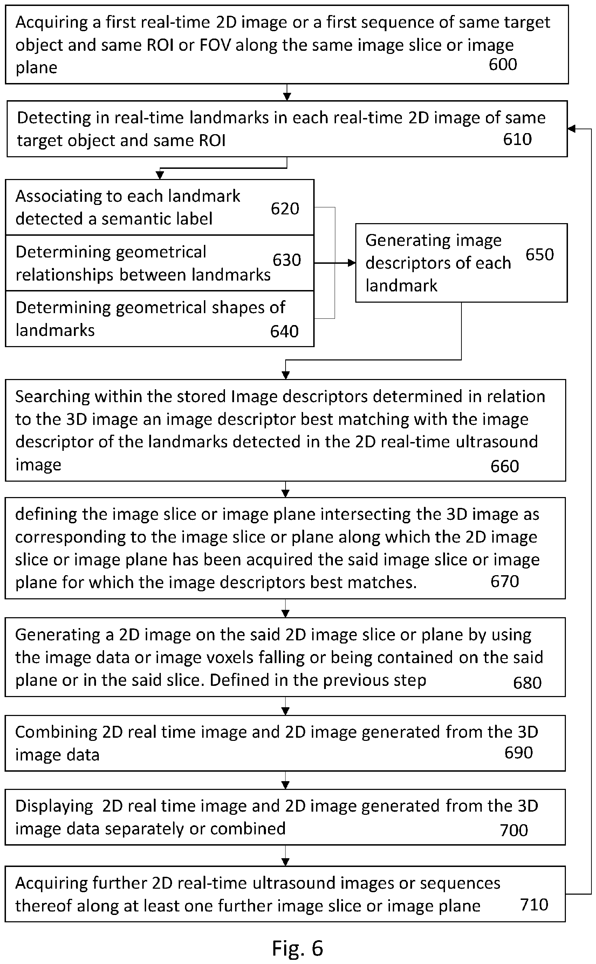

Figure 6 is a diagram showing a workflow of the process steps which are relating to the phase defined as "on-line" or real time phase.

Number 600 indicates the step of acquiring a first real-time two-dimensional image or a first sequence of images of a target object and/or of a ROI or FOV of a target object along an image slice or an image plane and which target object is the same one subjected to imaging at the step 500 by the tree-dimensional imaging technique. The acquired two-dimensional real-time image is immediately subjected to a step of detecting landmarks withing the said image as indicated by 610. The identified landmarks are analysed and univocally associated to specific semantic labels 620 and/or geometrical labels 630 and/or geometrical shapes and/or orientations of the said shapes 640 and these labels are recorded in a semantic description at step 650.

Once the semantic description of a two-dimensional, real-time image has been generated this data is used for carrying out a matching process with the semantic description of landmarks in the three-dimensional image among the ones generated at step 560 in the above disclosed embodiment of the off-line phase of the method. The said matching step is indicated by 660. Once a semantic description related to the three-dimensional image is found which matches the semantic description of the two-dimensional image generated at step 650, the image slice or plane corresponding to the said semantic description is considered as the one which position and orientation relatively to the target body and/or to a ROI or in a FOV of it corresponds or is registered with the image slice or plane along which the two-dimensional real-time image has been acquired as indicated at step 670. This image slice or image plane defined at step 670 is used for reconstructing along it a two-dimensional image using the data of the three-dimensional image which falls or are contained in the corresponding image slice or image plane as indicated at step 680. The two-dimensional image reconstructed at step 680 using the raw data and/or the image data and/or the image pixels or voxels contained in the said image slice or in the said image plane determined by the semantic description matching steps 670 and 680, may be followed by an image combination step of this image and of the two-dimensional, real-time image acquired at step 500. The combination step indicated with 690 is optional and can be carried out using different image combination algorithms.

According to an example the two images may be combine by simple superposition. According to a further embodiment combination may be obtained by using an algorithm generating the combined image by a linear or a nonlinear combination of the raw data and/or of the image data and/or of the parameters describing the appearance of the pixels or the voxels. The function may identical for the entire image or for the entire ROI or for the entire FOV or the function may be different for different regions of the said image and/or of the said ROI or of the said FOV. Further alternatives of image combination may be image fusion algorithms which are for example machine learning algorithms.

The two-dimensional images, i.e. the so defined Dynamic or real-time one and the static image may then be displayed at step 700 separately or as a combined image generated according one of the combination algorithms disclosed above in connection with step 690.

Since an imaging session carried out by acquiring real-time two-dimensional images by means of an ultrasound imaging apparatus may generate a sequence in time of two-dimensional images at a certain frame rate, the above disclosed steps 600 to 700 are normally repeated for each following image or for some of the said images as indicated by step 710.

According to the above method steps it is possible to provide a high definition and high quality three-dimensional image of the target body in combination with real-time images of the same target body which are used for monitoring time dependent events naturally occurring within the target body or caused by interventions on the said target body and which monitoring requires a rapid acquisition and image generation in order to follow the time development of the said event.

Rapidly registering the two-dimensional, real-time image acquired along a certain image slice or image plane with a corresponding image slice or plane intersecting the three-dimensional image of the target body and with the image reconstructed along this image slice or plane and generated using the information of the three-dimensional image allows to generate in real-time or in a quasi real-time condition a sequence of images which reproduces the image of the object using the high quality image information of the three-dimensional image and in which image the time development of the target body due to the time dependent event are shown using the information acquired in the two-dimensional real-time images. This information may be show combined such as embedded in the high quality or high-definition images reconstructed from the three-dimensional image.

## Claims

1. A method for tracker-less registering images of the same object generated from two different image datasets, and particularly from two different image datasets acquired by means of two different image acquisition methods and/or techniques, comprises:
- identifying landmarks in the image or images of the two datasets;
- associating to each landmark a univocal semantic description comprising semantic labels describing at least one feature of the landmark and/or geometrical labels describing at least one or some of the geometric relationships between the landmarks identified and/or geometrical labels relating to the shape and/or orientation of the said identified landmarks;
- considering two images of the said two data sets as being images along image slices or image planes registered one with the other when the semantic descriptions related to the landmarks present in the said images are matching one with the other.

2. Method according to claim 1 comprising the following steps:
a) providing an image data set for generating a three-dimensional image of a region of interest (ROI) in a target body;
b) processing the image data set with an anatomical landmark detector;
c) identifying each or some of the anatomical landmark in the said image and associating to the corresponding image data a semantic description of the anatomical landmark represented by the said image data;
d) saving the image data corresponding to each anatomical landmark and the associated semantic description of the corresponding anatomical landmark;
e) acquiring a first two-dimensional, real-time ultrasound image or a first sequence of two-dimensional, real-time images along at least one image slice or image plane of the same target body, the said image slice or image plane intersecting the same target body or the region of interest of the said target body as at step a);
f) processing said first two-dimensional, real-time image data or the said first sequence of real-time two-dimensional image data with an anatomical landmark detector for identifying anatomical landmarks within the said two-dimensional image data or the said sequence of two-dimensional image data acquired at steps e);
g) identifying each anatomical landmark within the said two-dimensional image data or within the said sequence of image data and associating to the corresponding image data a semantic description of the anatomical landmark represented by said image data;
h) determining the image slice or the image plane intersecting the said three-dimensional image which corresponds to the said image slice or the said image plane along which the said two-dimensional real-time image has been acquired at step e) by choosing the image slice or the image plane intersecting the said three-dimensional image data having the closest semantic description to the one associated at step g) to the image slice or the image plane along which the said first two-dimensional, real-time image or the said first sequence two-dimensional real-time images has been acquired at step e);
i) selecting the image data of the three-dimensional image data set falling on the image slice or image plane intersecting the said three-dimensional image data set determined at step h) and generating a two-dimensional image of the target body using the said selected image data.

3. Method according to claim 2, further comprising selecting all or at least some of the identified anatomical landmarks and the corresponding image data representing said selected anatomical landmarks and determining a geometrical relationship between said selected anatomical landmarks and the image slice or image plane along which the two-dimensional real-time image or the said sequence of two-dimensional real-time images has been acquired and in which the said selected anatomical landmarks has been identified.

4. Method according to claim 1 or 2, wherein the position and orientation of a two-dimensional image slice or plane is defined by said selected one or more predefined landmarks which are present in the image along the said image slice or image plane and by the geometric relationship between the said selected landmarks.

5. Method according to one or more of the preceding claims, wherein the orientation of the said two-dimensional image slice or image plane is additionally defined by determining the geometrical shape of the said selected landmarks.

6. Method according to one or more of the preceding claims, further comprising displaying the two-dimensional image of the target body generated at step i) together with the two-dimensional real-time ultrasound image.

7. Method according to one or more of the preceding claims, wherein, for each two-dimensional image slice or plane, a descriptor of the position and orientation of said image slice or plane relatively to the target body or to a ROI or FOV of the said target body is generated and univocally associated to said two-dimensional image slice or plane, said descriptor comprising the list of the denominations of the selected landmarks and the list of their geometrical relationship and/or their geometrical shapes in said image slice or plane, the registration of the image slice or image plane along which each of the real time images are acquired is compared with the descriptors of two-dimensional image slices or image planes intersecting the said three-dimensional image or a ROI or a FOV of the said three-dimensional image;
- the said descriptors comprising the list of the denominations of the selected landmarks present on each one of the said image slices or planes intersecting the three-dimensional image and the list of their geometrical relationship and/or their geometrical shapes in the corresponding image slice or plane;
- choosing the image slice or image plane intersecting said three-dimensional image or a ROI or a FOV thereof as the one corresponding to the position and orientation of the image slice or the image plane along which said two-dimensional real-time image is acquired when the descriptor univocally related to the image slice or image plane intersecting said three-dimensional image or a ROI or a FOV thereof corresponds to the descriptor of the image slice or the image plane of said two-dimensional real-time image.

8. Method according to claim 7, wherein the descriptors are compared by applying a matching algorithm such as a correlation algorithm or a machine learning algorithm such as a classification algorithm.

9. System for registering images of the same object generated from two different image datasets and particularly from two different image modality datasets comprising:
- at least a processing unit comprising at least a processor, memory, a data input interface for receiving a three-dimensional image data of a target object, a data output interface;
- a scanner in communication with the processing unit for acquiring real-time two-dimensional images of the target object or of a ROI or a FOV of such target object;
- a processing algorithm providing the instructions for making said at least one processing unit able to carry out the processing steps of the method according to any claim 1 to 8 for registering at least one of said real-time, two-dimensional images with an image generated along an image slice or plane intersecting said three-dimensional image and which position and orientation relatively to the target object or a ROI or a FOV thereof corresponds to the position and orientation of the image slice or image plane along which a corresponding one of the said two-dimensional real-time images has been acquired.

10. System according to claim 9, further comprising an image combination or fusion processor comprising a processing unit and a memory in which an image combination and/or fusion program is stored or may be stored, said combination and/or fusion program comprising the instructions for the processing unit which, when executed, renders said processing unit able to carry out the steps of combining and/or fusing a real-time two-dimensional image acquired by the scanner with a two-dimensional image generated with the contribution of the image data of the said three-dimensional image falling or contained in an image slice or on an image plane which position and orientation in relation to the target object or a ROI or a FOV thereof corresponds to the position and orientation of the image slice or the image plane along which the said real-time two-dimensional image has been acquired by said scanner.

11. System according to claim 9 or 10, further comprising:
at least one display and a display processor which comprises the instructions for displaying on the display the images acquired by the said scanner and the images generated from said three-dimensional image along an image slice or image plane which position and orientation in relation to the target object or a ROI or a FOV thereof corresponds to the position and orientation of the image slice or the image plane along which said real-time two-dimensional image has been acquired by said scanner;
said display instructions being configured for alternatively displaying said two images separated one form the other or one beside the other or said two images overlaid and/or combined and/or fused in an image comprising the image information of both said images.

12. System according to one or more of the preceding claims 9 to 11, wherein the scanner for acquiring real-time, two-dimensional images is an ultrasound imaging system.

13. System according to one or more of the preceding claims 9 to 12, wherein the system for acquiring three-dimensional images is an MRI system, a CT imaging system, a PET imaging system, a Fluorography imaging system.

14. System according to one or more of the preceding claims 9 to 13, **characterized in** being an imaging system for acquiring images of a target object and in which there is integrated a system for registering images of the same object generated from two different image datasets one of which datasets being the image data acquired by said imaging system and/or also a system for combining or fusing the registered or matched two-dimensional images acquired and generated in real time and the images generated with the contributions of the three-dimensional image falling or contained in the image slice or the image plane having matching orientations and positions relatively to the target object or a ROI or a FOV thereof as the image slice or image plane along which said two-dimensional real-rime images have been acquired according to claims 9 to 13 and carrying out a method according to claim 1 to 8.

15. System according to claim 14, **characterized in** being an ultrasound imaging system for acquiring real-time two-dimensional images.
